# EUROPEAN PATENT SPECIFICATION

(11) **EP 3 595 441 B1**
(45) Date of publication and mention of the grant of the patent: **02.08.2023**
(21) Application number: 18714180.9
(22) Date of filing: 16.03.2018
(51) Int. Cl.: A01N 25/02, A61P 33/00, A61K 31/7048, A01P 7/02, A01N 43/90, A61K 47/10, A61K 47/20, A61K 9/00, A61P 33/14

(54) **STABLE TOPICAL VETERINARY COMPOSITION**
STABILE TOPISCHE TIERMEDIZINISCHE ZUSAMMENSETZUNG
COMPOSITION VETERINAIRE TOPIQUE STABLE

(30) Priority: 17.03.2017 SI 201700085
(43) Date of publication of application: 22.01.2020
(73) Proprietor: KRKA, d.d., Novo mesto, 8000 Novo mesto (SI)
(72) Inventor: LESKOVAR, Denise, 2317 Oplotnica (SI); MIKLIC, Spela, 1000 Ljubljana (SI); KOLENC, Ivanka, 8000 Novo mesto (SI); CATOVIC, Alen, 8350 Dolenjske Toplice (SI); KINCL SKUBE, Maja, 8000 Novo mesto (SI)
(74) Representative: Hoefer & Partner Patentanwälte mbB
(86) International application number: PCT/EP2018/056667
(87) International publication number: WO 2018/167271

(56) References cited:
- EP-A1- 2 412 241
- EP-A1- 2 891 403
- EP-A2- 1 142 577
- WO-A1-00/30449
- WO-A1-2006/127487
- DATABASE WPI Week 201638 Thomson Scientific, London, GB; AN 2016-18492V XP002780810, -& CN 105 395 484 A (WUHAN HUISHENG BIOTECHNOLOGY CO LTD) 16 March 2016 (2016-03-16)
- .: "Revolution (Selamectin): MATERIAL SAFETY DATA SHEET", , 11 May 2004 (2004-05-11), pages 1-8, XP055472986, U.S.A. Retrieved from the Internet: URL:http://aycardovetcenter.com/downloads/ RevolutionMSDS.pdf [retrieved on 2018-05-07]
- .: "Revolution/Stronghold/Paradyne: Material Safety Data Sheet", , 24 September 2015 (2015-09-24), pages 1-13, XP55473023, U.S.A. Retrieved from the Internet: URL:https://www.zoetisus.com/contact/pages /product_information/msds_pi/msds/paradyne .pdf [retrieved on 2018-05-07]
- .: "CABI Bookshop: Macrocyclic Lactones in Antiparasitic Therapy by J. Vercruysse and R. Rew", , 1 December 2002 (2002-12-01), page 1, XP55473283, U.K. Retrieved from the Internet: URL:https://www.cabi.org/bookshop/book/978 0851996172 [retrieved on 2018-05-08]
- MAJELLA E. LANE: "Skin penetration enhancers", INTERNATIONAL JOURNAL OF PHARMACEUTICS, vol. 447, no. 1-2, 1 April 2013 (2013-04-01), pages 12-21, XP55171893, ISSN: 0378-5173, DOI: 10.1016/j.ijpharm.2013.02.040 cited in the application
- ADRIAN C. WILLIAMS ET AL: "Penetration enhancers", ADVANCED DRUG DELIVERY REVIEWS, vol. 64, 1 December 2012 (2012-12-01), pages 128-137, XP55054681, ISSN: 0169-409X, DOI: 10.1016/j.addr.2012.09.032 cited in the application

## Description

The present invention relates to a storage stable veterinary composition. More specifically, the invention discloses the storage stable veterinary antiparasitic composition comprising selamectin and a storage stable veterinary antiparasitic composition comprising selamectin for use as topical composition in treating, controlling and/or preventing of ectoparasite and endoparasite infections in warm-blooded animals.

### Background of the invention

Animals including domestic and farm animals are often susceptible to parasite infestations. In order to improve efficiency in animal handling there is a great need in efficient control of parasites in an animal and in prevention of any possible harmful effects on the treated animal.

The macrocyclic lactones such as for example avermectins and milbemycins and their derivatives are products of soil microorganisms belonging to the genus *Streptomyces.* The macrocyclic lactones have a potent, broad antiparasitic spectrum at low dose levels and can be used to treat infections of more than 300 species of endoparasites and ectoparasites in a wide range of hosts. The macrocyclic lactones are well absorbed when administered orally or parenterally while the pour-on and spot-on formulations exhibit greater variability. Regardless of the route of administration, macrocyclic lactones are extensively distributed throughout the body and concentrate particularly in adipose tissue.

Some of the avermectins in commercial use are ivermectin, abamectin, doramectin, eprinomectin, and selamectin and some of commercially available milbemycins are milbemycin oxime and moxidectin.

Selamectin with a chemical name 25-Cyclohexyl-4'-O-de(2,6-dideoxy-3-O-metyl-α-L-arabino-hexopyranosyl)-5-demetoxy-25-de(1-methylpropyl)-22,23-dihydro-5-(hydroxyimino)-avermectin A1a (USAN) is a semi-synthetic compound of the avermectin class. Selamectin was first disclosed in PCT publication No WO94/15944 as a potent 5-oximino monosaccharide for use as an antiparasitic agent, particularly for treatment of prophylaxis of flea Infestations.

Selamectin paralyses and/or kills a wide range of invertebrate parasites through interference with their chloride channel conductance causing disruption of normal neurotransmission. This inhibits the electrical activity of nerve cells in nematodes and muscle cells in arthropods leading to their paralysis and/or death. Selamectin has adulticidal, ovicidal and larvicidal activity against fleas. The composition marketed as Revolution^{®} and Stronghold^{®} is indicated for the treatment and prevention of flea infestations, for the prevention of heartworm disease and for the treatment of ear mites in cats and dogs. Additionally, the composition is used for the treatment of biting lice infestations (Felicola *subrostratus*), for the treatment of adult roundworms (*Toxocara cati*) and for the treatment of adult intestinal hookworms (*Ancylostoma tubaeforme)* in cats *and* in dogs for the treatment of biting lice infestations (*Trichodectes canis*), for the treatment of sarcoptic mange (caused by *Sarcoptes scabiei*) and for the treatment of adult intestinal roundworms (*Toxocara canis*).

The marketed composition is disclosed in PCT publication No WO2000/030449 as composition comprising (a) about 0.1-50% w/v selamectin ; (b) about 1-50% v/v a di (C₂₋₄ glycol) mono (C₁₋₄ alkyl) ether; (c) an optional antioxidant; and (d) an optional skin acceptable volatile solvent q. s. v/v. Particularly preferred glycol ether is dipropylene glycol monomethyl other (DPGMME). In the product information leaflet of the marketed composition it Is disclosed that stiff hair, clumping of hair, hair discoloration, or a slight powdery residue may be observed at the treatment site in some animals.

Several antiparasitic compositions comprising more than one active substance from different chemical classes are known. The veterinary antiparasite composition disclosed in PCT publication No WO2017/017091 comprises three different active substances fipronll, selamectin and praziquantel in an acceptable pharmaceutical vehicle that are represented by some glycol derivatives. US patent No US6991801 describes a spot-on combination composition comprising at least one macrocyclic lactone and at least one compound selected from praziquantel, morantel and pyrantel, a thickening agent, a liquid carrier vehicle comprising a solvent which could be DPGMME and a co-solvent and a crystallization inhibitor. PCT publication No WO2015/147655 discloses anthelmintic formulation of at least one macrocyclic lactone and levamisole wherein both active substances are solubilised together in a solvent system comprising dimethyl sulfoxide (DMSO) and at least one humectant. The concentration of DMSO is approximately 50 to 80% by weight.

EP 2 891 403 A1 discloses a soil nematode control emulsion in which a 16-membered ring macrolide compound is dissolved by using a water-soluble solvent having a boiling point of 150°C or higher, and a surfactant. WO 2006/127487 A1 describes a composition for topical administration which comprises on a weight to volume basis from about 0.1 to about 10% of a substantially water- insoluble anti-parasitic macrolide compound, about 5% to about 40% of metaflumizone; about 0% to about 15% of a penetrating agent; about 2 to about 8% of a surfactant; and about 50% to about 80% of a carrier solvent.

Several penetration enhancers have been described in the state of the art as being suitable for topical transfer of the active substance through the skin. It is well known that there is no universal penetration enhancer or vehicle that would be optimal for the delivery of all classes of topical drugs across the skin of all species. Selection of optimal vehicle for transdermal delivery of a drug requires a close match between the physical and chemical properties of the drug, vehicle and skin lipids of the particular animal.

DMSO, first synthesized in 1866, is used in a number of regulated products in human and veterinary medicine. DMSO is a highly polar aprotic substance knowing to enhance topical penetration of drugs owing to its ability to displace bound water from the stratum corneum. Literature describes the penetration enhancing activities of DMSO and its effectiveness as an excellent accelerator but this effect is concentration dependent. Increases in drug penetration have been reported with DMSO concentrations as low as 15%. However, as disclosed by A.C.Williams et al., Advanced Drug delivery Reviews 64 (2012) 128-137 and M.E.Lane, International Journal of Pharmaceutics 447 (2013) 12-21 the significant increase in permeability generally requires higher concentrations than 60%. At these relatively high concentrations DMSO can cause erythema and wheals of the stratum corneum and may denature some proteins. A further problem is the metabolite dimethyl sulphide produced from the solvent that produces a foul odour on the breath. On the other side DMSO has been shown to have bactericidal, bacteriostatic and fungistatic activity, anti-oxidative properties and it possesses mild to moderate neuroprotective effects.

Therefore, there is a need to prepare the stable veterinary antiparasitic composition comprising an antiparasitic active substance and DMSO present in such an amount that provides desirable penetration of the active substance through the skin and at the same time takes advantages of DMSO (such as bactericidal, bacteriostatic and fungistatic activity, anti-oxidative properties and neuroprotective effect) and at the same time minimizes the disadvantages of DMSO (such as erythema and wheals of the stratum corneum). Accordingly, it was an object of the present invention to provide a stable topical veterinary composition which is a skin-non-irritating and environmentally friendly and which may also contribute to an analgesic activity, a swelling-reducing activity and/or an anti-inflammatory activity. In addition to that, there was a need for a composition which would not cause stiffness of hair, clumping of hair, hair discoloration and powdery residue at the site of application.

This objective is surprisingly achieved by the storage stable topical veterinary composition comprising 0.5 - 30 % w/v of selamectin, DMSO in an amount between 0.5-15% of the composition, an organic solvent and optionally an antioxidant. The topical veterinary composition according to the present invention is a topical veterinary composition for use as a medicament for treating, controlling and/or preventing of ectoparasite and endoparasite infections in warm-blooded animals.

### Description of the invention

A first aspect of the present invention is directed to a storage stable topical veterinary composition comprising:
a) 0.5 - 30 % w/v selamectin ,
b) 0.5-15% w/v dimethyl sulfoxide (DMSO),
c) an organic solvent and
d) optionally an antioxidant.

According to another object or embodiment of the present invention a macrocyclic lactone, which is selamectin , is combined with another active substance suitable for veterinary application, such as active ingredients with parasiticidal activity selected from ectoparasiticides, endoparasiticide or endectocides. Ectoparasiticides can be selected from organochlorines, organophosphates, carbamates, amidines, synthetic pyrethroids, benzoylureas, juvenile hormone analogues, neonicotinoids, fhenylpyrazoles, spinosyns, isoxazolines. Endoparasiticides can be selected from benzimidazoles, imidazothiazoles, tetrahydropyrimidines, isoquinolines, salicylanilides.

The term "% wt. of the composition" can be used herein interchangeably with the term "% wt., based on the total weight of the topical veterinary composition". The term "DMSO in an amount between ... to ... % wt. of the composition" can be used herein interchangeably with the term "DMSO in an amount between ... to ... % wt., based on the total weight of the topical veterinary composition".

The organic solvent is an organic solvent other than DMSO.

Optionally, a topical veterinary composition of the present invention comprising selamectin may further comprise another active substance suitable for veterinary application, such as active ingredients with parasiticidal activity selected from ectoparasiticides, endoparasiticide or endectocides. Ectoparasiticides can be selected form organochlorines, organophosphates, carbamates, amidines, synthetic pyrethroids, benzoylureas, juvenile hormone analogues, neonicotinoids, phenylpyrazoles, spinosyns, isoxazolines. Endoparasiticides can be selected from benzimidazoles, imidazothiazoles, tetrahydropyrimidines, isoquinolines, salicylanilides.

Selamectin can be prepared by the routes of synthesis reported in the following references: US981500, EP677054, WO94015944, US6906184, EP1003764, WO99007721, EP2835376 wherein selamectin is prepared from doramectine. Selamectin can be used in crystalline, amorphous form or in the form of solvates. The preferred form is amorphous form.

The median particle size of selamectin used for the preparation of the composition of the present invention can be less than 200 µm. The particle size can be determined by laser diffraction methods using instruments such as Mastersizer 2000.

Purity grade of selamectin used in the composition of the present invention can be above 90%, preferably above 95%, more preferably above 98% as determined by HPLC. Preferably the specification for limits of related compounds comply with Ph. Eur. monograph for selamectin (especially complies with Ph. Eur. monograph version in force 17.03.2017).

The amount of selamectin present in the composition according to the present invention ranges from 0.5 to 30% w/v, preferably from 2 to 20% w/v, more preferably from 4 to 14% w/v, especially from 6 to 12% w/v. According to one embodiment, the amount of selamectin present in the composition according to the present invention can be 6 % w/v. According to another embodiment, the amount of selamectin present in the composition according to the present invention can be 12% w/v.

DMSO is present in the composition in an amount of 0.5 to 15% wlv, preferably in an amount of 2 to 14.5% w/v, more preferably in an amount of 4 to 14% w/v.

Ratio of selamectin to DMSO can range from 3 to 1, preferably from 2 to 1 and more preferably from 1.5 to 1.

According to one object or embodiment of the present invention an organic solvent can be selected from the group consisting of alcohols, ethers, esters, ketones, such as acetone, ethyl acetate, methanol, ethanol, isopropanol, preferably alcohols such as isopropyl alcohol, ethanol, and more preferably isopropyl alcohol is used as an organic solvent.

Alcohols can be in particular alcohols of formula R^{a}-OH, wherein R^{a} can be selected from alkyls, especially can be selected from C1-C6 alkyls (e.g. can be selected from C1 alkyl, C2 alkyl, C3 alkyls, C4 alkyls, C5 alkyls), such as straight chain C1-C6 alkyls, and branched C3-C6 alkyls. Ethers can be in particular ethers of formula R^{b}-O-R^{c}, wherein R^{b}, R^{c}, can be each independently selected from alkyls, especially can be each independently selected from C1-C4 alkyls. Esters can be in particular esters of formula R^{d}-C(O)-O-R^{e}, wherein R^{d}, R^{e}, can be each independently selected from alkyls, especially can be each independently selected from C1-C4 alkyls. Ketones can be in particular ketones of formula R^{f}-C(O)-R^{g}, wherein R^{f}, R^{g}, can be each independently selected from alkyls, especially can be each independently selected from C1-C4 alkyls.

The term "wlv" means "weight/volume". "1% w/v" means 1 g in 100 ml of the topical veterinary composition. Weight and volume, especially volume of the topical veterinary composition, can be in particular determined at a temperature of 20°C, especially at a temperature of 20°C and at a pressure 101325 Pa.

The topical veterinary composition can be a fluid (especially at a temperature of 20°C, e.g. at a temperature of 20°C and at a pressure 101325 Pa). The topical veterinary composition can be a solution or suspension (especially at a temperature of 20°C, e.g. at a temperature of 20°C and at a pressure 101325 Pa), preferably a solution (especially at a temperature of 20°C, e.g. at a temperature of 20°C and at a pressure 101325 Pa).

The amount of organic solvent present in the composition according to the present invention can range from 55% w/v to 99% w/v, preferably from 65% w/v to 95% w/v more preferably from 70% w/v to 90% w/v.

According to one object or embodiment of the present invention an antioxidant can be selected from the group consisting of butylated hydroxyanisole, butylated hydroxytoluene, tocopherols, ascorbic acid, sodium metabisulphite, propyl gallate, sodium thiosulphate or a mixture of not more than two of them, preferably butylated hydroxytoluene and butylated hydroxyanisole and more preferably butylated hydroxytoluene is used as an antioxidant. The amount of an antioxidant present in the composition according to the present invention can range from 0- 0.3% by weight, preferably 0.05-0.25% by weight, most preferably 0.05-0.15% by weight.

The present invention pertains also to the following formulation embodiments:
The composition embodiment 1 of the present invention is a storage stable topical veterinary composition comprising:
a) 0.5 - 30 % w/v of selamectin,
b) 0.5 -15% w/v of DMSO
c) an organic solvent and
d) optionally an antioxidant.

In particular, the organic solvent is an organic solvent other than DMSO. The organic solvent can be in particular selected from the group consisting of alcohols, ketones, ethers, esters, especially selected from the group of acetone, ethyl acetate, methanol, ethanol, and isopropanol.

The composition embodiment 2 of the present invention is a storage stable topical veterinary composition comprising:
a) 2 - 20 % w/v of selamectin ,
b) 2 -14.5 % w/v of DMSO
c) an organic solvent and
d) optionally an antioxidant.

In particular, the organic solvent is an organic solvent other than DMSO. The organic solvent can be in particular selected from the group consisting of alcohols, ketones, ethers, esters, especially selected from the group of acetone, ethyl acetate, methanol, ethanol, and isopropanol.

The composition embodiment 3 of the present invention is a storage stable topical veterinary composition comprising:
a) 4 -14 % w/v of selamectin,
b) 4-14 % w/v of DMSO
c) an organic solvent and
d) optionally an antioxidant.

In particular, the organic solvent is an organic solvent other than DMSO. The organic solvent can be in particular selected from the group consisting of alcohols, ketones, ethers, esters, especially selected from the group of acetone, ethyl acetate, methanol, ethanol, and isopropanol.

The composition embodiment 4 characterised in that the composition embodiments 1 to 3 comprise isopropyl alcohol as an organic solvent.

The composition embodiment 5 of the present invention characterised in that is comprises
a) 12 % w/v of selamectin,
b) 4-14 % w/v of DMSO
c) isopropyl alcohol and
d) optionally an antioxidant.

The composition embodiment 6 of the present invention characterised in that is comprises
a) 6 % w/v of selamectin,
b) 4 -14 % w/v of DMSO
c) isopropyl alcohol and
d) optionally an antioxidant.

The composition embodiment 7 of the present invention characterised in that it comprises
a) 6 - 12 % w/v of selamectin,
b) 4-14 % w/v of DMSO
c) isopropyl alcohol and
d) optionally an antioxidant.

The composition embodiment 8 of the present invention is a composition as defined in claim 1 and is a storage stable topical veterinary composition comprising:
a) 0.5 - 30 % w/v of selamectin,
b) DMSO in an amount between 0.5-15% wt. of the composition,
c) an organic solvent and
d) optionally an antioxidant.

In particular, the organic solvent is an organic solvent other than DMSO. The organic solvent can be in particular selected from the group consisting of alcohols, ketones, ethers, esters, especially selected from the group of acetone, ethyl acetate, methanol, ethanol, and isopropanol. The composition embodiment 9 of the present invention is a composition as defined in claim 1 and is a storage stable topical veterinary composition comprising:
a) 2-20 % w/v of selamectin,
b) DMSO in an amount between 2 -14.5 % wt. of the composition,
c) an organic solvent and
d) optionally an antioxidant.

In particular, the organic solvent is an organic solvent other than DMSO. The organic solvent can be in particular selected from the group consisting of alcohols, ketones, ethers, esters, especially selected from the group of acetone, ethyl acetate, methanol, ethanol, and isopropanol. The composition embodiment 10 of the present invention is a composition as defined in claim 1 and is a storage stable topical veterinary composition comprising:
a) 4 -14 % w/v of selamectin ,
b) DMSO in an amount between 4 -14 % wt. of the composition,
c) an organic solvent and
d) optionally an antioxidant.

In particular, the organic solvent is an organic solvent other than DMSO. The organic solvent can be in particular selected from the group consisting of alcohols, ketones, ethers, esters, especially selected from the group of acetone, ethyl acetate, methanol, ethanol, and isopropanol.

The composition embodiment 11 characterised in that the composition embodiments 8 to 10 comprise isopropyl alcohol as an organic solvent.

The composition embodiment 12 of the present invention characterised in that it comprises
a) 12 % w/v of selamectin,
b) DMSO in an amount between 4 -14 % wt. of the composition,
c) isopropyl alcohol and
d) optionally an antioxidant.

The composition embodiment 13 of the present invention characterised in that it comprises
a) 6 % w/v of selamectin,
b) DMSO in an amount between 4 -14 % wt. of the composition,
c) isopropyl alcohol and
d) optionally an antioxidant.

The composition embodiment 14 of the present invention characterised in that it comprises
a) 6 - 12 % w/v of selamectin,
b) DMSO in an amount between 4 -14 % wt. of the composition,
c) isopropyl alcohol and
d) optionally an antioxidant.

The composition according to the present invention may additionally comprise one or more veterinary acceptable excipients selected from the group consisting of co-solvents, stabilizers, anti-nucleating agents, crystallization inhibitor, spreading agents, systemic absorption agents and similar. A single excipient may have one or more functions.

Preferably, a composition according to the present invention, especially a spot-on composition according to the present invention, can be a fluid composition, in particular a liquid composition.

The composition according to the present invention can be administered at monthly intervals, particularly preferred is once monthly application.

The composition according to the present invention can be filled into containers for single dose application. Suitable containers are, for example, plastic tubes consisting of polypropylene, pipettes consisting of polypropylene or of a blister film (polypropylene/cyclicolefin-copolymer/ polypropylene) and a foil lidstock (aluminium/polypropylene co-extruded).

The tubes or pipettes may be sealed into secondary packaging such as clear PVC blister closed by heat sealing with aluminium foil, polychlorotrifluoroethylene PCTFE/PVC heat sealed blister packs or aluminium sachets in order to protect the formulation from moisture or evaporation. The composition of the present invention may be filled into primary packaging material and packed into secondary packaging under inert gas atmosphere. Optionally nitrogen or argon can be used as inert gas atmosphere in the packaging procedure, wherein nitrogen is especially preferred.

The compositions according to the invention can be prepared by conventional processes by mixing the active compounds under stirring with the other components and preparing a solution. The process according to the present invention comprises the following steps:
- adding organic solvent and optionally antioxidant into a mixer
- (optionally) adding other veterinary acceptable excipient(s) into solvent mixture under stirring until clear solution is obtained
- adding selamectin to the mixture under stirring until clear solution is obtained
- adding dimethyl sulfoxide to the mixture under stirring until clear solution is obtained.

The process is preferably carried out in an inert atmosphere, i.e. under flow of inert gas, such as nitrogen, argon, helium, neon, krypton, xenon, preferably nitrogen or argon, most preferably nitrogen.

Adding other veterinary acceptable excipient(s) can be in particular adding one or more veterinary acceptable excipients selected from the group consisting of co-solvents, stabilizers, anti-nucleating agents, crystallization inhibitor, spreading agents, and systemic absorption agents.

In particular, the process for preparing a composition of the present invention can comprise the following steps:
a) adding organic solvent and optionally antioxidant into a mixer;
b) (optionally) adding one or more other veterinary acceptable excipient(s) into solvent mixture (obtained after step a)) under stirring until clear solution is obtained, e.g. said one or more other veterinary acceptable excipients can be selected from the group consisting of co-solvents, stabilizers, anti-nucleating agents, crystallization inhibitor, spreading agents, and systemic absorption agents;
c) adding selamectin to the mixture (obtained after step a) or after optional step b)) under stirring until clear solution is obtained; and
d) adding dimethyl sulfoxide to the mixture (obtained after step c)) under stirring until clear solution is obtained.

According to another object or embodiment of the present invention the composition is for use in the topical application to the skin of an animal in the form of liquid composition, preferably in the form of spot-on or pour-on composition, more preferably in the form of spot-on composition.

According to another object or aspect of the present invention the composition is a composition for use in treating, controlling and/or preventing of ectoparasite and endoparasite infections in warm-blooded animals. More particularly, the composition is a composition for use in the treatment and prevention of flea infestations, for use in the prevention of heartworm disease, for use in the treatment of ear mites, for use in the treatment of biting lice infestations, for use in the treatment of adult roundworms, for use in the treatment of adult intestinal hookworms, for use in the treatment of biting lice infestations, for use in the treatment of sarcoptic mange and for use in the treatment of adult intestinal roundworms.

According to a further aspect of the present invention, a topical veterinary composition of the present invention is for use as a medicament.

According to a still further aspect of the present invention, a topical veterinary composition of the present invention can be for use as a medicament for treating, controlling and/or preventing of ectoparasite and endoparasite infections, especially in warm-blooded animals. In particular, a topical veterinary composition of the present invention can be for use as a medicament for treating, and controlling ectoparasite and endoparasite infections, especially in warm-blooded animals. Furthermore, a topical veterinary composition as disclosed herein can be for use as a medicament for preventing ectoparasite and endoparasite infections, preferably in warm-blooded animals.

According to one object or embodiment of the present invention the treated warm-blooded animal can be selected from the group consisting of warm-blooded animals, such as e.g. household animals (especially pet animals) and laboratory animals, especially e.g. dogs, cats, hedgehogs, ferrets, horses, sheep, and rodents, such as gerbils, hamsters, chinchillas, fancy rats and guinea pigs, preferably dogs, cats, ferrets, guinea pigs, and more preferably dogs and cats.

By the composition of the present invention several problems has been solved.

The composition according to the present invention has shown surprisingly good cosmetic profile. During the in-vivo study significantly less stiffness and clamping of hair at the application site was observed for the composition of the present invention than for the reference product. White residue in the form of oily patches at the application site was observed in much lesser extent than for the reference product. This characteristic may be attributed to the fact that DMSO is rapidly absorbed skin penetrant and that it has the ability to carry active ingredients through membranes thereby provides for rapid absorption of active ingredients.

DMSO has anti-oxidative properties and provides for stabilization of the active ingredients against oxidative decomposition, such as selamectin , thus compositions according to present invention retain their therapeutic activity and their specific properties over a longer period of time. The inventors have shown that the composition according to present invention is surprisingly storage stable. Stability testing under accelerated conditions (40°C, 60% RH) has shown that the total content of related substances was not increased after several months of storage.

The above listed advantages provided by a composition of the present invention comprising DMSO may contribute to increasing the treatment acceptance of subjects (e.g. animals, especially pet and farm animals) treated with a composition of the present invention and the treatment acceptance of the care givers, e.g. pet owners, keepers in laboratories and animal shelters. An increased treatment acceptance of subjects will in turn largely facilitate the time-intensive work of treating subjects (e.g. pet animals) with a composition of the present invention for control of parasites. Treatment acceptance would encourage the care givers to treat animals on a regularly basis thus providing long term prevention against parasites.

The bioequivalence of the composition of the present invention with the marketed product has been shown in the in-vivo study.

The present invention is illustrated using the following non-limiting examples.

### Examples

### Example 1

12%w/v composition

| Ingredient | Content [mg/ml] |
|---|---|
| Isopropyl alcohol | 619.7 |
| Butylated hydroxytoluene | 0.8 |
| Selamectin | 120 |
| Dimethyl sulfoxide | 112.56 |
| Total weight | 853.06 |

### Example 1a

### Bioequivalence study

The formulation was tested in vivo bioequivalence study on dogs. The formulation was demonstrated to be bioequivalent to the reference STRONGHOLD^{®} (Selamectin ) 12% w/v Spot-on Solution for Dogs, since the Test to Reference ratio of geometric LS means as well as the corresponding 90% confidence interval for Cₘₐₓ and AUC₀₋ₜ of selamectin were all within the acceptance range of 80.00 to 125.00%.

### Example 1b

### Stability data:

The composition according to Example 1 was stable under room and accelerated stability testing conditions, which was shown in the following Table:

| Sample | Testing condition | Total Impurities (%) |
|---|---|---|
| 12% w/v spot on solution | t₀ | ≤ 0.30% |
| | 25°C/60% RH - 18m | ≤ 0.30% |
| | 40°C/75% RH - 6m | ≤ 0.30% |

| | | |
|---|---|---|
| m - months RH - relative humidity | | |

The analytical method used for determining related substances of selamectin was a reversed-phase gradient HPLC method, using a 4 µm C18 column or a suitable alternative. The mobile phase was a combination of water and acetonitrile, with UV detection at 243 nm.

### Example 2

6%w/v composition

| Ingredient | Content [mg/ml] |
|---|---|
| Isopropyl alcohol | 700.32 |
| Butylated hydroxytoluene | 0.8 |
| Selamectin | 60 |
| Dimethyl sulfoxide | 56.28 |
| Total weight | 817,4 |

### Example 2a

### Stability data:

The composition according to Example 2 was stable under room and accelerated stability testing conditions, which was shown in the following Table:

| Sample | Testing condition | Total Impurities (%) |
|---|---|---|
| 6% w/v spot on solution | t₀ | ≤ 0.30% |
| | 25°C/60% RH - 12m | ≤ 0.30% |
| | 40°C/75 % RH - 6m | ≤ 0.30% |

| | | |
|---|---|---|
| m - months RH - relative humidity | | |

The analytical method used for determining related substances of selamectin was a reversed-phase gradient HPLC method, using a 4 µm C18 column or suitable alternative. The mobile phase was a combination of water and acetonitrile, with UV detection at 243 nm.

### Example 2b

### Bioequivalence study

The formulation was tested in vivo bioequivalence study on cats. The formulation was demonstrated to be bioequivalent to the reference STRONGHOLD^{®} (Selamectin ) 6% w/v Spot-on Solution, since the Test to Reference ratio of geometric LS means as well as the corresponding 90% confidence interval for Cₘₐₓ and AUC₀₋ₜ of selamectin were all within the acceptance range of 80.00 to 125.00%.

## Claims

1. A storage stable topical veterinary composition comprising:
a) 0.5 - 30 % w/v of selamectin,
b) 0.5 -15% w/v of DMSO
c) an organic solvent and
d) optionally an antioxidant.

2. The storage stable topical veterinary composition according to claim 1 comprising:
a) 2 - 20 % w/v of selamectin,
b) 2 -14.5 % w/v of DMSO
c) an organic solvent and
d) optionally an antioxidant.

3. The storage stable topical veterinary composition according to claim 2 comprising:
a) 4 - 14 % w/v of selamectin,
b) 4-14 % w/v of DMSO
c) an organic solvent and
d) optionally an antioxidant.

4. The storage stable topical veterinary composition according to any of the preceding claims **characterised in that** isopropyl alcohol is used as an organic solvent.

5. The storage stable topical veterinary composition according to any of the preceding claims **characterised in that** it comprises
a) 12 % w/v of selamectin ,
b) 4 -14 % w/v of DMSO
c) isopropyl alcohol and
d) optionally an antioxidant.

6. The storage stable topical veterinary composition according to any of the preceding claims **characterised in that** it comprises
a) 6 % w/v of selamectin,
b) 4-14 % w/v of DMSO
c) isopropyl alcohol and
d) optionally an antioxidant.

7. The storage stable topical veterinary composition according to any of the preceding claims **characterised in that** it is in the form suitable for spot-on application.

8. The storage stable topical veterinary composition according to any of the preceding claims **characterised in that** it is a fluid.

9. The storage stable topical veterinary composition according to any of the preceding claims **characterised in that** it is a solution or suspension.

10. The storage stable topical veterinary composition according to any one of claims 1 to 9 for use as a medicament.

11. The storage stable topical veterinary composition according to any one of claims 1 to 9 for use as a medicament for treating, controlling and/or preventing of ectoparasite and endoparasite infections, especially in warm-blooded animals.

12. The storage stable topical veterinary composition for use as a medicament for treating, controlling and/or preventing of ectoparasite and endoparasite infections according to claim 11 in warm-blooded animals.

13. The storage stable topical veterinary composition according to claim 11 for use as a medicament for treating, and controlling ectoparasite and endoparasite infections, especially in warm-blooded animals.

14. The storage stable topical veterinary composition according to claim 11 for use as a medicament for preventing ectoparasite and endoparasite infections, especially in warm-blooded animals.

## Patentansprüche

1. Lagerstabile topische tiermedizinische Zusammensetzung, umfassend:
a) 0,5 - 30 % (w/v) Selamectin,
b) 0,5 - 15 % (w/v) DMSO
c) ein organisches Lösungsmittel und
d) gegebenenfalls ein Antioxidans.

2. Lagerstabile topische tiermedizinische Zusammensetzung gemäß Anspruch 1, umfassend:
a) 2 - 20 % (w/v) Selamectin,
b) 2 - 14,5 % (w/v) DMSO
c) ein organisches Lösungsmittel und
d) gegebenenfalls ein Antioxidans.

3. Lagerstabile topische tiermedizinische Zusammensetzung gemäß Anspruch 2, umfassend:
a) 4 - 14 % (w/v) Selamectin,
b) 4 - 14 % (w/v) DMSO
c) ein organisches Lösungsmittel und
d) gegebenenfalls ein Antioxidans.

4. Lagerstabile topische tiermedizinische Zusammensetzung gemäß einem der vorhergehenden Ansprüche, **dadurch gekennzeichnet, dass** Isopropylalkohol als ein organisches Lösungsmittel verwendet wird.

5. Lagerstabile topische tiermedizinische Zusammensetzung gemäß einem der vorhergehenden Ansprüche, **dadurch gekennzeichnet, dass** sie umfasst
a) 12 % (w/v) Selamectin,
b) 4 - 14 % (w/v) DMSO
c) Isopropylalkohol und
d) gegebenenfalls ein Antioxidans.

6. Lagerstabile topische tiermedizinische Zusammensetzung gemäß einem der vorhergehenden Ansprüche, **dadurch gekennzeichnet, dass** sie umfasst
a) 6 % (w/v) Selamectin,
b) 4 - 14 % (w/v) DMSO
c) Isopropylalkohol und
d) gegebenenfalls ein Antioxidans.

7. Lagerstabile topische tiermedizinische Zusammensetzung gemäß einem der vorhergehenden Ansprüche, **dadurch gekennzeichnet, dass** sie in der Form, welche für Spot-on-Verabreichung geeignet ist, vorliegt.

8. Lagerstabile topische tiermedizinische Zusammensetzung gemäß einem der vorhergehenden Ansprüche, **dadurch gekennzeichnet, dass** sie ein Fluid ist.

9. Lagerstabile topische tiermedizinische Zusammensetzung gemäß einem der vorhergehenden Ansprüche, **dadurch gekennzeichnet, dass** sie eine Lösung oder Suspension ist.

10. Lagerstabile topische tiermedizinische Zusammensetzung gemäß einem der Ansprüche 1 bis 9 zur Verwendung als ein Medikament.

11. Lagerstabile topische tiermedizinische Zusammensetzung gemäß einem der Ansprüche 1 bis 9 zur Verwendung als ein Medikament zur Behandlung, Kontrolle und/oder Vorbeugung von Ektoparasiten- und Endoparasiteninfektionen, insbesondere bei warmblütigen Tieren.

12. Lagerstabile topische tiermedizinische Zusammensetzung zur Verwendung als ein Medikament zur Behandlung, Kontrolle und/oder Vorbeugung von Ektoparasiten- und Endoparasiteninfektionen gemäß Anspruch 11 bei warmblütigen Tieren.

13. Lagerstabile topische tiermedizinische Zusammensetzung gemäß Anspruch 11 zur Verwendung als ein Medikament zur Behandlung und Kontrolle von Ektoparasiten- und Endoparasiteninfektionen, insbesondere bei warmblütigen Tieren.

14. Lagerstabile topische tiermedizinische Zusammensetzung gemäß Anspruch 11 zur Verwendung als ein Medikament zur Vorbeugung von Ektoparasiten- und Endoparasiteninfektionen, insbesondere bei warmblütigen Tieren.

## Revendications

1. Composition vétérinaire topique stable au stockage comprenant :
a) 0,5 à 30% p/v de sélamectine,
b) 0,5 à 15% p/v de DMSO
c) un solvant organique et
d) éventuellement un antioxydant.

2. Composition vétérinaire topique stable au stockage selon la revendication 1 comprenant :
a) 2 à 20% p/v de sélamectine,
b) 2 à 14,5% p/v de DMSO
c) un solvant organique et
d) éventuellement un antioxydant.

3. Composition vétérinaire topique stable au stockage selon la revendication 2 comprenant :
a) 4 à 14% p/v de sélamectine,
b) 4 à 14% p/v de DMSO
c) un solvant organique et
d) éventuellement un antioxydant.

4. Composition vétérinaire topique stable au stockage selon l'une quelconque des revendications précédentes, **caractérisée en ce que** l'alcool isopropylique est utilisé comme solvant organique.

5. Composition vétérinaire topique stable au stockage selon l'une quelconque des revendications précédentes, **caractérisée en ce qu'**elle comprend
a) 12% p/v de sélamectine,
b) 4 à 14% p/v de DMSO
c) l'alcool isopropylique et
d) éventuellement un antioxydant.

6. Composition vétérinaire topique stable au stockage selon l'une quelconque des revendications précédentes, **caractérisée en ce qu'**elle comprend
a) 6% p/v de sélamectine,
b) 4 à 14% p/v de DMSO
c) l'alcool isopropylique et
d) éventuellement un antioxydant.

7. Composition vétérinaire topique stable au stockage selon l'une quelconque des revendications précédentes, **caractérisée en ce qu'**elle se présente sous la forme appropriée pour une application cutanée localisée.

8. Composition vétérinaire topique stable au stockage selon l'une quelconque des revendications précédentes, **caractérisée en ce qu'**elle est un fluide.

9. Composition vétérinaire topique stable au stockage selon l'une quelconque des revendications précédentes, **caractérisée en ce qu'**elle est une solution ou une suspension.

10. Composition vétérinaire topique stable au stockage selon l'une quelconque des revendications 1 à 9 pour une utilisation comme médicament.

11. Composition vétérinaire topique stable au stockage selon l'une quelconque des revendications 1 à 9 pour une utilisation comme médicament pour le traitement, la lutte contre et/ou la prévention d'infections à ectoparasites et à endoparasites, en particulier chez les animaux à sang chaud.

12. Composition vétérinaire topique stable au stockage pour une utilisation comme médicament pour le traitement, la lutte contre et/ou la prévention d'infections à ectoparasites et à endoparasites selon la revendication 11 chez les animaux à sang chaud.

13. Composition vétérinaire topique stable au stockage selon la revendication 11 pour une utilisation comme médicament pour le traitement et la lutte contre des infections à ectoparasites et à endoparasites, en particulier chez les animaux à sang chaud.

14. Composition vétérinaire topique stable au stockage selon la revendication 11 pour une utilisation comme médicament pour la prévention d'infections à ectoparasites et à endoparasites, en particulier chez les animaux à sang chaud.
